# EUROPEAN PATENT APPLICATION

(11) **EP 4 541 298 A1**
(43) Date of publication of application: **23.04.2025**
(21) Application number: 24207376.5
(22) Date of filing: 18.10.2024
(51) Int. Cl.: A61B 17/88, A61F 2/46

(54) **SURGICAL TOOL SYSTEM WITH A SMART SCREWDRIVER**

(30) Priority: 18.10.2023 BE 202305867
(71) Applicant: Surgical Sensors bv, 9100 Sint-Niklaas (BE)
(72) Inventor: DELPORT, Hendrik, 9100 Sint-Niklaas (BE); DELPORT, Filip, 9100 Sint-Niklaas (BE); GHIJSELINGS, Ignace, 9100 Sint-Niklaas (BE); VAN ONSEM, Stefaan, 9100 Sint-Niklaas (BE); PHILPOTT, Matthew, 9100 Sint-Niklaas (BE); CLEMENT, Eli, 9100 Sint-Niklaas (BE)
(74) Representative: Brantsandpatents bv

(57) **Abstract**

The present invention relates to a surgical tool system, to support prosthetic surgery for determining an optimal balance point of the ligamentous complex or ligament capsule of a joint, based on and visualized in a real-time stress-strain curve (SS curve) of the ligaments, comprising: a screwdriver suitable for determining stress and strain, comprising: two or more sensors, a motor, a coupling element, a control unit, suitable for reading out a torque exerted by the coupling element and an executed rotation angle of the coupling element measured by the sensors and forwarding them to an interface; the interface on which software runs, suitable for reading out a torque exerted by the coupling element and an executed rotation angle of the coupling element measured by the sensors, and interpreting and forwarding them to an interface suitable for visually displaying a stress value and strain value in an SS curve.

## Description

### TECHNICAL FIELD

The invention relates to the technical field of medical/surgical devices, systems and methods. More specifically, the invention relates to a surgical tool system, a screwdriver and a method for supporting prosthetic surgery, including on knees, shoulders, hips, but also other joints.

### PRIOR ART

Total knee replacement surgery (TKR), also called total knee arthroplasty (TKA) or total knee prosthesis surgery (TKP), is becoming an increasingly important treatment for chronic knee pain and joint dysfunction. Techniques for placing knee prostheses must be optimized to offer a high likelihood of good outcomes and to reduce prosthesis wear, thus reducing the need for repeated TKA surgeries, also known as revision surgery. Improved techniques and devices are therefore beneficial for all TKA patients, with improved knee joint function, longer life of the prosthetic knee and a more natural feel for the patient.

The knee is generally defined as the point of articulation of the femur with the tibia. Structures that make up the knee include the distal thighbone (femur), the proximal shinbone (tibia), the kneecap (patella), and the ligamentous and other soft tissues in and around the knee joint. Four ligaments are particularly important in the functioning of the knee: the anterior cruciate ligament (ACL), the posterior cruciate ligament (PCL), the inner or medial collateral ligament (MCL), and the outer lateral collateral ligament (LCL). These ligaments work together to maintain stability of the knee and prevent it from bending or twisting too far.

In an arthritic knee, the protective cartilage in the knee joint has worn away, leading to an inflammatory and mechanical process that causes pain and discomfort. The primary goals of a TKA procedure are to replace the distal end of the femur, the proximal end of the tibia, and often the inner surface of the patella with prosthetic parts to prevent bone-on-bone contact and provide smooth, well-aligned surfaces for joint movements, while also creating a stable knee joint that moves through a wide range of motion.

Knee replacement surgery is a relatively recent procedure, with the first modern interventions dating back to the 1970s, and is therefore still undergoing major evolution. Although the results have improved over the years, 20% of people remain dissatisfied after having their implant placed. There are several reasons for this, including the patients' expectations, but certainly also many surgical-technical issues (as yet uncontrolled variables). One of the problems for which there is no clear solution yet is balancing the ligaments in and around the knee during the procedure.

The collateral ligaments, which connect the distal femur and the proximal tibia on the medial and lateral aspects of the knee, are responsible for much of the knee's stability and movement. If one of the collateral ligaments is too lax or too tight relative to the other collateral ligament, the knee will usually be unstable, range of motion may be limited, the patella may track incorrectly, and the femur and/or tibia may wear unevenly, leading to inflammatory responses and pain. Uneven ligament tension after TKA surgery usually causes joint instability and poor patellar tracking, limited range of motion and decreased knee function, as well as uneven, increased wear of the implant, often requiring repeat surgery. Therefore, it is necessary for the short and long term success of a TKA procedure to achieve a balanced ligament tension in the knee in order to obtain a well-functioning knee. Proper soft tissue balance is a very important factor for patient satisfaction.

There are a number of surgical devices known, for example US9743971 concerns a programmable screwdriver and method for inserting screws into bone are disclosed. The screwdriver contains a torque sensor for measuring the torque during screw insertion, a rotational motion sensor for measuring the rotation of the screwdriver, and a microprocessor. Whenever a surgeon turns a screw to be secured into the bone, the torque sensor measures the torque and sends this information to the microprocessor. Once a predetermined torque is reached, the microprocessor begins measuring the next rotation of the screwdriver up to a predetermined rotation limit, sending a signal to alert the surgeon to stop tightening.

DE102016102298 concerns a tool device, in particular for a surgical tool, a surgical tool with a tool device, a system with a display unit and a surgical tool and a method for operating a tool device.

US2006243464 concerns an apparatus and a method for measuring torque and angular displacement. More specifically, some embodiments relate to an apparatus and method for measuring an annular tension between two anatomical structures based on measurements of the torque and angular displacement.

EP2073726 concerns apparatus and a method for performing knee replacement surgery, wherein a spacer is inserted between the femur and the tibia while the patella is in place, said spacer separating the femur from the tibia by an amount substantially equal to or greater than the required flexion gap, and wherein an alignment device is used to perform femoral bone cuts, said device being temporarily attached to a mounted tibial plate.

While the surgical devices mentioned can certainly be valuable in performing various types of surgical procedures, they may not be optimal for facilitating positioning of the prosthesis during a TKA procedure. In this procedure it is crucial to achieve an optimal balance point, which requires accurate and precise measurements. Furthermore, these aids do not specifically focus on the positioning of a prosthesis. Furthermore, these known techniques have failed to produce reproducible results in the hands of orthopedic surgeons.

Patients often complain that their operated knee does not feel "normal," possibly due to a disruption of the proprioceptive mechanism of the knee, which may explain the persistence of postoperative dissatisfaction. This feeling may be a result of a slight instability or overstrained knee. Conversely, conventional alignment goals can achieve mechanically sound bony alignment while ignoring or damaging soft tissues. This known positioning of TKA components often generates technically uncorrectable collateral ligament imbalance. Furthermore, planning is often performed on static models that provide detailed anatomical information but no functional information.

There remains a need for an improved method to optimize TKA placement. The present invention aims to find a solution for at least some of the above problems.

### SUMMARY OF THE INVENTION

In a first aspect, the invention relates to a surgical tool system according to claim 1. Preferred embodiments of the first aspect of the invention are described in claims 2 to 6.

The invention is, among other things, applicable to knee surgery. During a TKA procedure, it is important to balance the medial and lateral structures of the knee and ensure a balanced and symmetrical flexion/extension gap. This sometimes requires that the soft tissues of the knee be released and repeated bone resection performed to achieve proper positioning (which is not desirable). Achieving a balanced knee therefore requires more than just removing the worn cartilage, bone and soft tissues but requires a careful and detailed assessment of the positioning and balance of the prosthesis during the TKA procedure.

A balanced knee is of great importance for patient satisfaction after a TKA procedure. Correct handling of soft tissues is therefore an important factor for a successful outcome in primary TKA. These soft tissues will guide the bony resections, thus ensuring a well-balanced knee (without damaging the ligaments). To this end, the surgical tool system offers an advantageous solution for determining the optimal balanced point within and between the lateral and medial compartments of the knee during TKA surgery. With this advantageous surgical tool system, a zone, and preferably the exact point, is defined where the ligaments and ligament complex need to be balanced individually. An algorithm interprets the stress-strain curve recorded by the tool and provides the surgeon with predefined zones of laxity, as well as the optimal balanced position for the medial and lateral sides separately. The tool can automatically stop in a predetermined zone. The surgeon can ignore the suggested automatic stopping point if desired and interpret the stress-strain curve according to their balancing philosophy. The surgeon may then decide to implant the femoral component in a tighter or looser position. Instead of using a manual screwdriver, where the surgeons determine this subjectively, an automated and instrumented screwdriver is used.

Other applications include shoulder surgery, hip surgery, but the invention is in principle applicable to any joint. In what follows, the invention is explained primarily in its application to knee surgery, but the principles are readily translatable by those skilled in the art to surgical procedures on other joints.

The surgical tool system uses one or more sensors that measure the stress and strain, a motor that drives the coupling element and a control unit that reads the data and forwards it to an interface. The surgical tool system includes software capable of interpreting the measured data and visually displaying it in a real-time stress and strain curve (stress-strain curve or SS-curve) of the ligaments. This provides real-time insight into the stress and strain of the ligaments, allowing surgeons to determine an optimal balance point and reduce the risk of complications during and after surgery. The tool system is compatible with interfaces such as computers or mobile devices and can therefore be easily accessible to surgeons in various clinical settings. In addition, it reduces complexity relative to current approaches, as it simplifies the process of attaching a measuring device to the patient and does not incorporate parts of a measuring device into the prosthetic components. The system therefore offers a very advantageous solution for optimizing a TKA procedure, whereby an optimal balance between the soft tissues, in particular the optimal balance point within and between the MCL and LCL, is obtained.

The use of the described surgical tool system offers many benefits for prosthetic knee surgery. First, the system provides an objective and accurate tool to determine soft tissue balance, which reduces the risk of complications during and after surgery and improves patient satisfaction. In addition, using the surgical tool system reduces the complexity of current approaches and simplifies their handling. The system is modular and does not need to be incorporated into the prosthetic components, which increases the efficiency of the procedure. In addition, the system offers the possibility to use artificial intelligence to determine an optimal zone, and preferably the optimal balance point for each patient, resulting in a more personalized and effective treatment. The surgical tool system can be used in conjunction with any implant philosophy. It is flexible and can be integrated as a possible add-on to existing placement systems, such as robots, navigation or other advanced technologies.

In a second aspect, the invention relates to a screwdriver for use in orthopedic surgery. The screwdriver comprises:
- a coupling element for connecting the screwdriver to a transcondylar pin;
- one or more sensors, comprising at least one encoder suitable for measuring a performed rotation angle of the coupling element;
- a motor suitable for driving the coupling element;
- a control unit, suitable for reading out a measured or calculated torque exerted by the coupling element and an executed rotation angle of the coupling element and transmitting them to an interface.

Preferably, the screwdriver comprises an activation element suitable for controlling the motor to rotate the coupling element via a gearbox.

Preferably, the coupling element that is aligned with the housing is equipped with bearings, making it suitable for rotating around a single axis.

Preferably, the screwdriver comprises at least one slip ring suitable for transmitting signals from the torque sensor to the control unit.

Preferably, the software stores a stress value and a strain value and links these to patient data.

The screwdriver provides an objective tool to perform ligament balancing. An important aspect of the screwdriver is the built-in sensors, which measure the stress and strain of soft tissues, such as ligaments, in real time. These measurements allow the surgeon to better understand the loads placed on the soft tissues and make the necessary corrections to optimize balance. Another important aspect of the screwdriver is the built-in motor, which can drive the coupling element and the sensors. This makes the rotation angle more accurate and consistent than manual tightening by a surgeon, resulting in better control over the applied forces and rotation angle, thereby improving the accuracy and efficiency of the measurements. It helps reduce the variability caused by the surgeon's senses. It helps the surgeon to make more accurate measurements and better control the applied forces and rotation angles, resulting in better efficiency and precision during surgery. In other words, it ensures higher reproducibility and reduces variability between different surgeons or even within the same surgeon. Furthermore, the motor helps reduce fatigue and pain in the surgeon's hands, wrists and arms, reducing the risk of injury.

The screwdriver has a control unit that allows the surgeon to analyze and control the measurements from the sensors during the operation, further improving efficiency and precision and reducing human error. In addition, the screwdriver is a standalone, portable tool that can operate completely wirelessly, increasing mobility and flexibility during use and making it easy to use in different operating rooms without additional cabling.

In a third aspect, the invention relates to a method for determining an optimal balance point of a ligamentous complex or ligament capsule of a joint, such as for example the lateral and medial compartments of the knee, according to claim 7. Preferred embodiments of the second aspect of the invention are described in claims 8 to 15.

The method described is aimed at determining an optimal zone, and preferably the optimal balance point of the lateral and medial compartments of the knee when placing a knee prosthesis, or of the ligamentous complex in other joints. This is done by visualizing a real-time stress-strain curve (SS curve) of the ligaments, which is generated using a surgical tool system. An algorithm interprets the stress-strain curve recorded by the tool system and provides the surgeon with predefined zones of laxity, and preferably the optimal balanced position for the medial and lateral sides separately. The tool system, including the screwdriver, can automatically stop in a predetermined zone. The surgeon can ignore the suggested automatic stopping point if desired and interpret the stress-strain curve according to their balancing philosophy. They can then decide to implant the femoral component into a tighter or looser position.

The procedure begins with placing a transcondylar pin into the distal end of the femur and a condylar nut at the distal end of the pin. Next, a tibial baseplate is placed at the proximal end of the tibia. A screwdriver is placed on the transcondylar pin through a coupling element, and the screwdriver is then driven by a motor to apply a torque to the coupling element, causing the transcondylar pin to rotate. By measuring the rotation of the transcondylar pin and using the screw thread information, the gap distance between the femur and tibia can be determined and ultimately converted to a strain value.

The advantages of this approach are numerous. First, it can increase the safety of the procedure because the forces exerted on the ligaments during placement of the prosthesis can be measured and controlled, which can help prevent damage to the ligaments and other tissues. This can lead to a faster recovery and fewer complications after the procedure. Secondly, it can improve the patient's quality of life, as optimal balance and mobility of the implant will lead to less pain and improved functionality of the joint. This is due to better positioning of the implant. This may enable the patient to return to daily activities and hobbies that may have been difficult before. Thirdly, this approach may help reduce muscle fatigue by reducing the need for compensatory mechanisms, reduce implant loosening and subluxation, maintain balance of the soft tissues around the knee, and reduce implant wear.

These values (data) can be saved and processed later to arrive at the ideal values. They can also be communicated to an assisting robot or navigation system.

### DESCRIPTION OF THE DRAWINGS

**Figure 1** shows a perspective view of the screwdriver showing the built-in components according to an embodiment of the present invention.
**Figure 2** shows a cross-section of the screwdriver according to an embodiment of the present invention.
**Figure 3** shows a front view of the tool system of an embodiment of the present invention.
**Figure 4** shows a front view of the tool system of an embodiment of the present invention.

### DETAILED DESCRIPTION

The invention concerns a surgical tool system, a screwdriver and a method for supporting prosthetic surgery.

Unless otherwise defined, all terms used in the description of the invention, including technical and scientific terms, have the meaning as commonly understood by a person skilled in the art to which the invention pertains. For a better understanding of the description of the invention, the following terms are explained explicitly.

In this document, "a" and "the" refer to both the singular and the plural, unless the context presupposes otherwise. For example, "a segment" means one or more segments.

When the term "around" or "about" is used in this document with a measurable quantity, a parameter, a duration or moment, and the like, then variations are meant of approx. 20% or less, preferably approx. 10% or less, more preferably approx. 5% or less, even more preferably approx. 1% or less, and even more preferably approx. 0.1% or less than and of the quoted value, insofar as such variations are applicable in the described invention. However, it must be understood that the value of a quantity used where the term "about" or "around" is used, is itself specifically disclosed.

The terms "comprise," "comprising," "consist of," "consisting of," "provided with," "have," "having," "include," "including," "contain," "containing" are synonyms and are inclusive or open terms that indicate the presence of what follows, and which do not exclude or prevent the presence of other components, characteristics, elements, members, steps, as known from or disclosed in the prior art.

Quoting numeric intervals by the endpoints includes all integers, fractions, and/or real numbers between the endpoints, including those endpoints.

In the context of this document, the following terms are used interchangeably: "prosthesis" and "implant."

The terms "optimal balance point", "balance point", "optimal point" or "exact point" are synonymous and should be understood as the point at which the joint gap on the medial and lateral sides is equal over the entire arc of motion and at which the tension on the ligamentous complexes (MCL and LCL) is equal. It is the position wherein where the tension on the ligaments and the pressure distribution in the joint are most favorable, which ensures a stable and well-functioning joint, such as a knee, after surgery. Reaching this point is an important goal when placing implants to maximize the durability and success of the surgery.

The term "optimal zone" or "optimal balance zone" should be understood as a predefined zone of laxity. The optimal zone refers to the specific range on the stress-strain curve, determined by the algorithm, in which the soft tissues of the joint are most favorably balanced. Within this zone, the load on (e.g., the medial and lateral sides of) the joint is optimally distributed, resulting in a stable and well-functioning joint.

The acronyms "MCL" and "LCL" stand for "medial collateral ligament" and "lateral collateral ligament" respectively. These terms belong to anatomical terminology, which refers to the main ligaments of the knee. In the context of the document, the terms "MCL" and "LCL" are interchangeable. Furthermore, the term "ligament" refers to one of these ligaments, i.e., the medial collateral ligament or the lateral collateral ligament. If the anterior cruciate ligament (ACL) and posterior cruciate ligament (PCL) are present and preserved, they should preferably also be balanced. This means that when we use the term "ligament" it can refer to the MCL, the LCL, the ACL, or the PCL.

Ligaments are strong, fibrous structures in the body that connect bone to bone and help provide movement and stability to joints such as the knee, shoulder, hip, etc. When an external force is applied to the body, such as when a person bends his or her knee, it can lead to stress and strain on the ligaments in the knee.

The term "stress" refers to the amount of load placed on a ligament in response to an external force. It is a measure of the force per unit area exerted on the ligaments. For example, when a person bends his or her knee, stress is placed on the ligaments in the knee to support and stabilize the joint.

The term "strain" refers to the amount of deformation that occurs in the ligaments as a result of the stress. It is a measure of the amount of stretch the ligaments undergo in response to the stress. Excessive loading can lead to excessive strain or damage of the ligaments of the joint.

In a first aspect, the invention relates to a surgical tool system, to support prosthetic surgery of ligaments in or around a joint to determine an optimal balance point within the ligamentous complex or ligament capsule of the joint, based on and visualized in a real-time stress-strain curve (SS curve) of the ligaments, comprising:
- a screwdriver suitable for determining stress and strain, comprising:
   i. one or more sensors, comprising at least one encoder;
   ii. a coupling element, suitable for placing the screwdriver on an element to be measured;
   iii. a motor suitable for driving the coupling element, and optionally other sensors such as a torque sensor (if present);
   iv. a control unit, capable of determining a torque exerted by the coupling element and an executed rotation angle of the coupling element and forwarding them to an interface;
- the interface on which software runs, suitable for reading out the torque exerted by the coupling element and an executed rotation angle of the coupling element, interpreting and forwarding them to an interface, suitable for visually displaying a stress value and strain value in an SS curve, which is suitable for determining an optimal zone, and preferably an optimal point, wherein the ligament is balanced.

In particular, the system is suitable for knee surgery, wherein an optimal balance point of the lateral and medial compartments of the knee is determined. Other applications include hip surgery, shoulder surgery but also surgery on other joints. In what follows, the operation of the invention in all its aspects is mainly outlined with reference to knee surgery, but for those skilled in the art this description is easily transferable to application to other joints.

This tool system offers, among other things, a very advantageous solution for optimizing a TKA procedure, with the aim of obtaining an optimal balance between the soft tissues, in particular the optimal balance point of the MCL and LCL. This can be achieved thanks to the ability of the surgical tool system to generate an SS curve in a fast, accurate and simple way, wherein during rotation of the coupling element of the screwdriver, both the stress and the strain of the soft tissues, more specifically of the ligaments, are measured simultaneously. This allows the surgeon to visualize the stress and strain in real time on an interface, allowing the surgeon to determine the balance point of the lateral and medial compartments of the knee. This is crucial for prosthetic knee surgery, as it forms the basis for determining the correct placement and orientation of the knee prosthesis.

To ensure accurate measurements of the stress and strain, the screwdriver is equipped with, among other things, one or more sensors and a motor, while the coupling element ensures that the screwdriver makes a strong connection with, for example, a transcondylar pin. This reduces the chance of errors during measurement and ensures a more accurate measurement of the stress and strain. The control unit reads the measurements from the sensors and forwards them to the interface, where software interprets the data and generates the SS curve. This allows the surgeon to visualize the stress and strain in real time and determine the balance point of the lateral and medial compartments of the knee.

An important advantage of the described system is that it provides real-time insight into the stress and strain of the ligaments, allowing surgeons to determine an optimal zone, and preferably an optimal balance point, and reduce the chance of complications during and after surgery. Moreover, the interface of the system is compatible with both computers and mobile devices, making it easily accessible to surgeons in different clinical environments. The described system can therefore make an important contribution to improving the quality of prosthetic knee surgery and reducing the risks of complications and reoperations.

An SS curve, also known as a stress-deformation curve, of ligaments describes the relationship between the amount of stretch (strain) and the resulting force (stress) needed to elongate the ligaments. When a ligament is exposed to an increasing load, the strain of the ligament will increase, causing the force required to stretch the ligament further to also increase.

The initial rise of the curve is often called the linear region, wherein small loads lead to small strains in the ligament. This linear region represents the normal operating region of the ligament, wherein small loads do not cause permanent damage and the ligament is able to return to its original shape once the load is removed. As the loading increases, the ligament can reach its maximum strain and begin to deform. This is the area where the ligament loses its elasticity and is no longer able to fully return to its original shape after the load is removed. This is the area where the ligament can sustain permanent damage. Eventually, the ligament reaches its breaking point, the point at which the force required to stretch the ligament further can no longer be absorbed by the ligament and it tears. The breaking point is represented by the highest point on the curve, wherein the stress is maximum and the strain increases sharply.

Knowing the SS curve of ligaments is important for assessing the health of the ligaments and determining the risks of injury at different levels of loading. The SS curve of the ligaments in the knee will provide information on the ideal balance point of the knee.

If the curve of the ligament is observed, it is noted that there is a maximum load wherein the ligament retains its elasticity and returns to its original shape when the load is removed. This point is called the "elastic limit." When the ligament is further stressed, it reaches a point where it begins to deform and can no longer fully recover to its original shape. This point is called the "yield point." Ideally, the balance point of the knee should be above the elastic limit of the ligaments, but below the yield point. This means that the knee is stable and functions well under normal loads, but there is also sufficient stretch and resilience in the ligaments to accommodate powerful movements and sudden movements without getting damaged.

If the balance point of the knee is too low and below the elastic limit of the ligaments, this can lead to instability of the knee and an increased risk of ligament damage. If the balance point of the knee lies too high and above the yield point of the ligaments, this can lead to overloading and damage to the ligaments. Therefore, it is important to optimize the balance point of the knee for prosthesis placement. If the prosthesis is not properly aligned, this can lead to unequal stress on the ligaments and unnatural movement patterns, which can result in pain, dysfunction, and even the loosening of the prosthesis.

In a preferred embodiment, the surgical tool system further comprises a condylar pin and/or a condylar nut and/or a tibial plate. The condylar nut is suitable for attaching to an end of the femur and is suitable for receiving the transcondylar pin. The transcondylar pin is equipped with a thread. The transcondylar pin will be connected via the thread to a condylar nut, which transcondylar pin is suitable for exerting a force on the tibial plate. The tibial plate is suitable for protecting the tibia against the force exerted by the transcondylar pin and suitable for receiving the transcondylar pin.

In a preferred embodiment, the tool system comprises a threaded transcondylar pin. This is designed to attach securely and safely to the condylar nut and to provide optimal stability. The thread can have different shapes, such as a V-shaped thread, a trapezoidal thread, or a square thread. In addition, the thread can also be equipped with a self-locking system to prevent the pin from coming loose during measurements.

Preferably, the transcondylar pin of the surgical tool system can be made from any material with suitable compressive strength that meets the requirements for biocompatibility of surgical instruments. Such materials are, for example, stainless steel and titanium. Preferably, the transcondylar pin is biodegradable, in which case it may be made of biodegradable alloys (e.g., magnesium alloys) or polymers (e.g., polyglycolide (PGA), polylactide (PLA), poly(epsilon-caprolactone), poly(dioxanone), poly(lactide-co-glycolide), or a combination thereof. Further or alternatively, the materials may be provided with a ceramic coating. The head of the transcondylar pin, which is adapted to receive the coupling element of the screwdriver, can take various shapes, such as a round end face, a square end face, or a hexagonal end face. The shape of the end face is adapted to the shape of the coupling element of the screwdriver, so that they fit well together and the force can be transmitted effectively. The spacers can be made of.

In a preferred embodiment, the tool system comprises a condylar nut, which condylar nut includes internal threading. The condylar nut is suitable to be attached to one end of the femur, more specifically the femoral condyles. The nut contains internal threading and is therefore suitable to receive the transcondylar pin. Due to the screw connection between the transcondylar pin and the condylar nut, a force is exerted on the tibial plate, which will increase the gap between the tibia and femur and allow the stress-strain curve of the ligaments to be accurately measured. An advantage of the condylar nut is that it provides a simple and reliable way to position the transcondylar pin and the internal threading allows for precise adjustment and control of the force on the tibial plate.

Preferably, various forms of the condylar nut are possible, for example: A condylar nut with a special coating or finish to reduce friction and improve durability. A condylar nut with a larger or smaller diameter, to match the specific dimensions of the patient. A condylar nut with multiple internal threads, to optimize force distribution. It is important to consider factors such as strength, durability, sterilization, and ease of use in the design of the condylar nut.

Preferably, a traditional trapezoidal thread flank is used as the thread of the condylar nut. A possible alternative is the use of a square thread. This type of screw has square thread flanks instead of the traditional trapezoidal thread flanks. Additionally, there are various types of threads that can be used, such as metric, UNC, UNF or ACME thread. The choice of a certain type of thread depends on various factors, such as the application, the required strength and the desired ease of use.

In a preferred embodiment, the tool system comprises a tibial plate. A tibial plate is a plate that is placed on top of the tibia and will be used to protect the tibia from the force exerted by the transcondylar pin. The tibial plate forms a platform to receive the transcondylar pin. There are preferably different shapes and sizes of tibial plates possible, depending on the specific application and the patient's anatomy. For example, the tibial plate can be designed to be attached across the entire surface of the tibial plateau, thus distributing pressure and load over a larger area. Another possibility is that the tibial plate only covers the portion of the tibial plateau that will receive the transcondylar pin. The surface of the plate can be smooth but preferably has a surface similar to that of a golf ball, with dimples. In terms of material, the tibial plate can be made of stainless steel, titanium or polymethyl methacrylate (PMMA), PEEK (polyetheretherketone), materials with ceramic coatings, cobalt-chromium alloys, various polymers or other materials that can be used in the human body.

In a preferred embodiment, the surgical tool system comprises a transcondylar pin and a condylar nut and a tibial plate. The condylar nut and the transcondylar pin work together as part of the surgical tool system to exert a force on the tibial plate. The transcondylar pin is provided with a thread and is connected to the condylar nut which has an internal thread. When the transcondylar pin is screwed into the condylar nut, a force is exerted on the tibial plate. The force applied to the tibial plate depends on the direction and magnitude of the force applied to the condylar nut by the screwdriver. As the screwdriver is turned clockwise, the condylar nut will move up the thread of the transcondylar pin, applying a force to the tibial plate which will increase the distance between the tibia and femur. A good fit between the threads of the transcondylar pin and the internal thread of the condylar nut is essential to ensure proper force transmission. In addition, the shape of the end face of the transcondylar pin must be well matched to the shape of the coupling element of the screwdriver to allow good transmission of the rotational movement.

In a preferred embodiment, the interface comprises a computer or mobile device. Alternatively, the interface could be one of the following: a smartphone, a tablet, a watch, a microcontroller-based computing device, and the like. The interface on which software runs is suitable for reading out a torque exerted by the coupling element and an executed rotation angle of the coupling element measured by the sensors, and interpreting and forwarding them to an interface suitable for visually displaying a stress value and strain value in an SS curve. The interface offers several advantages for prosthetic knee surgery. Visualization of the SS curve provides surgeons with a clear picture of the stress and strain distribution in the knee, which can help determine the optimal balance point of the lateral and medial compartment of the knee during prosthetic knee surgery. The interface will display data in real-time, giving surgeons greater insight into the condition of the knee and enabling them to make rapid decisions and adjustments during surgery. In addition, the interface will be very user-friendly, which will improve the efficiency and accuracy of the surgical procedure. The interface can also store data for future use and reference, which can be valuable for evaluating the results of the surgical procedure and developing new strategies and techniques for knee prosthetic surgery.

In a second aspect, the invention relates to a screwdriver for use in orthopedic surgery, comprising a coupling element connecting the screwdriver to a transcondylar pin; one or more sensors, comprising at least one encoder suitable for measuring a performed rotation angle of the coupling element; a motor suitable for driving the coupling element; and a control unit, suitable for reading out and transmitting to an interface a measured or calculated torque exerted by the coupling element and an executed rotation angle of the coupling element.

This screwdriver is a special tool for use in orthopedic surgery, as it has a number of unique features that contribute to improved accuracy, efficiency and safety during surgery, preferably during a TKA procedure.

One of the most interesting features of this screwdriver is its ability to measure the stress and strain of soft tissues, such as ligaments, in real time. This is made possible by the built-in sensors, preferably including a torque sensor that measures the torque exerted on the coupling element, and an encoder that measures the rotation angle of the coupling element. These measurements allow the surgeon to gain a better understanding of the loads placed on the soft tissues.

Another important feature of this screwdriver is the built-in motor, which can drive the coupling element and the sensors. This makes the rotation angle more accurate and consistent than manual tightening by a surgeon, resulting in better control over the applied forces and rotation angle, thereby improving the accuracy and efficiency of the measurements. In addition, using a motor reduces fatigue and pain in the surgeon's hands, wrists and arms and reduces the risk of injury. When using a motorized screwdriver, a continuously controlled rotation can be applied compared to manual screwing. Thus, it completely avoids the need to manually tighten a screwdriver, which has many disadvantages.

A third important feature of this screwdriver is the presence of a control unit, with which the measurements from one or more sensors can be read and forwarded to an interface. This allows the surgeon to analyze and check the data during surgery, further improving efficiency and precision.

Finally, this screwdriver is a standalone, portable tool that can operate completely cordless, increasing mobility and flexibility during use. The device can be easily taken to different operating rooms and can be used quickly without the need for additional cabling.

In a preferred embodiment, the screwdriver has a coupling element provided with a hexagonal recess or protrusion that connects the screwdriver to preferably a transcondylar pin.

A hexagonal connection, also known as a six-sided connection, is a type of connection wherein one component has a hexagonal recess and the other component has a six-sided protrusion that fits perfectly into the recess. This ensures a tight, snug, and stable connection of the two components.

When the screwdriver is placed on the transcondylar pin, an optimal torque transfer is guaranteed by using a hexagonal connection, which increases the accuracy of the measurements. The sturdy connection also ensures that the screwdriver cannot unintentionally shift during measurements, increasing the reliability of the measurements. Play between the two is eliminated because hexagonal protrusions fit exactly into the hexagonal recesses. A solid connection between both elements is crucial for obtaining accurate measurements.

Alternatively, there are various forms of the coupling element that are suitable for creating a firm connection between the screwdriver and the transcondylar pin. For example, the coupling element may have a round recess combined with a round protrusion on the transcondylar pin. This results in a round connection that is also stable and close-fitting. Furthermore, a square connection, where the coupling element has a square recess or protrusion is a good alternative. Another option is to opt for a cross-shaped connection. Here the coupling element has a cross-shaped recess or protrusion, which can be useful when using the screwdriver in a hard-to-reach place. The cross shape allows the screwdriver to be turned in different directions to transfer the correct force. A tooth-shaped connection is also an option. In this case the coupling element has a tooth-shaped recess or protrusion, wherein the teeth interlock to create a strong connection. This can be useful when the screwdriver is used in a high-vibration environment, as the teeth ensure that the screwdriver remains firmly in place. The type of connection chosen depends on the specific application and requirements. Other shapes that create a strong connection are also possible.

In a preferred embodiment, the screwdriver has one or more sensors, at least one of which is a torque sensor suitable for measuring a torque exerted on the coupling element. Alternatively, the torque applied can also be calculated by measuring the characteristics of the power delivered to the motor, such as the electrical current and voltage. It should be noted, however, that this method is less accurate than measuring with a torque sensor. The advantage of measuring the torque with a torque sensor is that it is a very accurate and direct measurement of the force exerted on the coupling element.

The applied torque is optimally measured using the torque sensor and can then be converted into a force value, for example of ligaments. This force value can in turn be converted into a stress value, which can then be used in an SS curve. This curve can be interpreted by a surgeon to determine whether the force exerted on the tissue is within an acceptable range. The highly accurate measurement of the applied torque on the coupling element using a torque sensor is of crucial importance in the medical world due to the high precision required for surgical procedures. A small error in the torque measurement can lead to incorrect force and stress values, which can affect the interpretation of the results and potentially have harmful consequences for the patient. By using a torque sensor, a highly accurate measurement can be performed, leading to more reliable and accurate results and thus increased patient safety during the procedure.

In a preferred embodiment, the screwdriver has one or more sensors, at least one of which is an encoder suitable for measuring an executed rotation angle of the coupling element. Preferably a rotary encoder is used because a rotary encoder can accurately measure the rotation angle of the coupling element, and this information can be used to calculate the gap between the tibia and the femur. This is done by combining the pitch of the screw with the rotation angle to calculate the total displacement of the coupling element. By comparing the original gap between the tibia and femur with the calculated gap between the femur and tibia, a strain value of the ligaments can be determined. This is important because it gives an indication of the stress exerted on the ligaments during surgery. By using the encoder, this strain value can be calculated in a very accurate way.

Alternatively, various types of encoders are available that can be used to measure the rotation angle of the coupling element. In addition to rotary encoders, for example, linear encoders and angle encoders can be used. Linear encoders measure linear displacement and can be used to measure the axial movement of the screwdriver. Angle encoders measure the angle of a rotating object and can be used to measure the rotation angle of the coupling element.

In a preferred embodiment, the screwdriver has a control unit, suitable for reading out a torque exerted by the coupling element and an executed rotation angle of the coupling element measured by the sensors, and transmitting them to an interface. The screwdriver's control unit includes a processor and other essential electronic components placed on a PCB, making the whole thing function as a Printed Circuit Board Assembly (PCBA). This PCBA is used to process the signals from various sensors, such as the measured torque of the coupling element and the executed rotation angle of the coupling element, and transmits them to an interface. These signals can be transmitted to the control unit either wired or wirelessly, depending on the implementation of the wireless communication. An expert in this field can identify the appropriate hardware required to implement wireless operation, such as transmitters, receivers, and other hardware. The signals can then be displayed on a display unit, such as a personal computer or other processor, for display of the signals.

In a preferred embodiment, the screwdriver comprises a sterilizable housing. There are various materials suitable for sterilizable housings for the screwdriver, including stainless steel, aluminum, plastics, and titanium. Stainless steel is a durable material that is resistant to autoclaving and chemical disinfection methods and has good mechanical properties and corrosion resistance. Aluminum is lightweight, corrosion resistant and durable and can be anodized to protect the surface. Some plastics, such as polycarbonate, polyethylene and polypropylene, are suitable for sterilization, although not all types are suitable. Titanium is lightweight, corrosion-resistant and biocompatible and is often used in the medical field.

The choice of material depends on the specific sterilization methods used, durability and safety for use in a medical environment. The use of a sterilizable housing for the screwdriver in the described system offers several advantages. First of all, it ensures better hygiene in the operating room, because the tools can be thoroughly cleaned and sterilized between operations to prevent the spread of pathogens. In addition, using a sterilizable screwdriver housing can extend the life of the instrument by protecting it from wear and damage. This can save costs in the long run because it needs to be replaced less often. Finally, the sterilizable housing can improve the ease of use and ergonomics of the screwdriver. A well-designed housing will improve grip and reduce surgeon fatigue, which can result in more accurate stress and strain measurements and better tool performance during surgery.

In a further or alternative preferred embodiment, the screwdriver includes a visual indicator. If the screwdriver is equipped with a visual indicator, it can provide information about the stress and strain of the ligaments during the surgical procedure. For example, the visual indicator can use a color code to let the surgeon know when the stress and strain fall within a certain optimal range. Alternatively, the indicator can also provide a visual representation of the SS curve of the ligaments, similar to the interface running on the computer. This allows the surgeon to receive real-time feedback on the status of the ligaments during the procedure, without having to constantly look at a computer screen. For example, the information can be transferred to the visual indicator on the screwdriver via wireless communication with the control unit that receives and interprets the data from the sensors. This provides a seamless and efficient way to provide the relevant information to the surgeon during surgery.

In a preferred embodiment, the screwdriver will have a built-in power supply to power the sensors, motor, and other elements within the screwdriver. The power supply may consist of a battery, such as a rechargeable lithium-ion battery, built into the screwdriver. Another option is to connect the screwdriver to an external wired power supply, such as a mains power adapter. Having a built-in power supply offers the advantage of allowing the screwdriver to operate independently without the need to be connected to an external power supply, making the screwdriver more mobile and versatile. The battery can also be recharged so that the screwdriver can be reused without having to constantly provide new batteries.

In a preferred embodiment, the screwdriver includes an actuation element, such as a button, switch, pressure sensor, or the like, which is adapted to actuate the motor and rotate the torque sensor and the torque element via a gearbox. When the surgeon activates the actuation element, for example by pressing a button, the motor will be actuated immediately. The motor then drives the torque sensor and the coupling element via preferably a gearbox, causing them to rotate.

In a preferred embodiment, the screwdriver has at least one slip ring, which is suitable for sending signals from the torque sensor to the control unit. This offers the advantage that the screwdriver can be used without the sensors and electronics on the screwdriver needing to be connected with wires. This makes the screwdriver more mobile and easier to use during surgical procedures. Alternative solutions to using a slip ring include wireless communication via Bluetooth or Wi-Fi, but this can lead to delays or interference in signal transmission and requires good security to protect patient privacy.

In a preferred embodiment, the coupling element is aligned with the housing and provided with bearings, making it suitable for rotation about one axis. The bearings allow the coupling element to rotate smoothly, allowing for accurate measurements. Ball bearings are preferably used because they have very low friction and therefore consume little energy. This reduces the load on the screwdriver's battery or power supply and extends the tool's life.

In a further or alternative embodiment, the torque sensor and the coupling element are aligned with the housing and provided with bearings, allowing them to rotate about a single axis. The bearings allow the torque sensor and coupling element to rotate smoothly, enabling accurate measurements. Ball bearings are preferably used because they have very low friction and therefore consume little energy. This reduces the load on the screwdriver's battery or power supply and extends the tool's life.

In a third aspect, the invention relates to a method for determining an optimal balance point of the lateral and medial compartment of the knee, based on and visualized in a real-time stress-strain curve (SS curve) of the ligaments for positioning a knee prosthesis.

Again, this aspect can be varied for other joints, to find an optimal balance point for the ligamentous complex or ligament capsule thereof.

The advantageous method provided by the present invention aims to optimize the positioning of a knee prosthesis in the patient's joint and thus provide a number of advantages. This invention provides an advantageous method for optimizing the positioning of a knee prosthesis within the patient's joint, with the aim of obtaining an optimal balance between the soft tissues, in particular the optimal balance point of the MCL and LCL. The method uses a surgical tool system that can generate an SS curve in a fast, accurate and simple manner, wherein during the rotation of the coupling element of the screwdriver both the stress and the strain are measured simultaneously. This allows the surgeon to visualize the stress and strain of both the medial and lateral compartments in real time on an interface, allowing the surgeon to determine the optimal point, both medially and laterally, on the SS curve. This optimal point determines the position of the implant.

In addition, this method will also increase the safety of the procedure, because the forces exerted on the ligaments during placement of the prosthesis can be measured and controlled. This will help prevent damage to the ligaments and other tissues, which can lead to a faster recovery and fewer complications after the procedure. In addition, this method can contribute to a better quality of life for the patient, because optimal balance and mobility of the implant will lead to less pain and improved functionality of the joint. This may enable the patient to return to daily activities and hobbies that may have been difficult before.

In a first step, the method involves placing a transcondylar pin in the distal end of the femur, placing a condylar nut at the distal end of the transcondylar pin, and placing a tibial baseplate at a proximal end of the tibia. In this way, a stable basis is created for the further steps in the method. The aforementioned placement of the transcondylar pin, condylar nut and tibial baseplate provides a reliable basis for further measurements.

In a next step of the method, the screwdriver is placed on the transcondylar pin via a coupling element. The coupling element connects the screwdriver to the transcondylar pin, allowing the screwdriver to be positioned in a precise and stable manner. This provides a reliable measurement of the stress and strain of the ligaments in the knee.

Preferably, the coupling element is placed onto the transcondylar pin via a hexagonal connection before attaching the screwdriver. This provides a strong and stable connection between the screwdriver and the transcondylar pin, eliminating any play between the two as the hexagonal protrusions fit precisely into the hexagonal recesses. A solid connection between both elements is crucial for obtaining accurate measurements. By using a hexagonal connection, optimum transfer of force and torque is guaranteed, which increases the accuracy of the measurements. The sturdy connection also ensures that the screwdriver cannot unintentionally shift during measurements, increasing the reliability of the measurements.

Alternatively, the screwdriver can be placed on the transcondylar pin via a square connection, wherein the coupling element of the screwdriver has a square recess and the transcondylar pin has a square protrusion that fits exactly into the recess, or vice versa. This connection also provides a strong and secure connection between the screwdriver and the transcondylar pin, but may not be as stable as a hexagonal connection. Other possible connections include triangular connections or round connections. Another possible connection is a clamp connection, where a clamp or clip is used to secure the screwdriver to the transcondylar pin. This can be a quick and easy solution, but it may be less strong and stable than a hexagonal or square connection. A further alternative is a magnetic connection, wherein a magnet is used to attach the screwdriver to the transcondylar pin. This can be useful because it can be quickly and easily applied and removed, but it can be less secure than other connections and can be susceptible to magnetic interference during measurements.

When choosing a connection, it is important to consider factors such as strength, stability and reliability of measurements. A hexagonal connection is preferred because it ensures a firm and stable connection without play, resulting in reliable and accurate measurements.

After placing the screwdriver on the transcondylar pin via the coupling element, the screwdriver is driven by a motor, whereby the coupling element and preferably also a torque sensor are rotated. By applying the applied torque via the coupling element on the screwdriver to the transcondylar pin, it will be rotated. The motor will ensure that the movements of the screwdriver, more specifically the rotation of the torque sensor and the torque element, can be carried out very precisely and in a controlled manner. This makes the measurements more reliable and accurate. The torque sensor in the screwdriver provides the means to measure the torque applied on the coupling element, although, as mentioned, this can also be derived from information about the power supplied to the motor during operation. This allows the torque applied to the coupling element, and therefore also to the transcondylar pin, to be accurately recorded and controlled. This is essential for obtaining reliable and accurate measurements. The use of the coupling element and motor allows the transcondylar pin to be rotated with precision and control. This rotation of the transcondylar pin allows the range of motion of the joint to be measured. This is of great importance for obtaining reliable and accurate measurements.

In a preferred embodiment, the motor is driven after the actuation element has been activated. This actuation element can be a trigger, button, switch or something similar. When the screwdriver trigger is activated, the motor will start working. The torque of the motor is transferred to the torque sensor and the torque element of the screwdriver via a gearbox.

In a preferred embodiment, the applied torque is measured by the torque sensor. The torque sensor is built into the screwdriver and records the torque applied to the coupling element. The torque sensor in the screwdriver measures the torque applied to the coupling element and passes this information on to the control unit so that the measurements can be recorded accurately. Using the torque sensor also allows to check whether the force with which the screwdriver is driven remains constant, which increases the reliability of the measurements. Alternatively, the torque applied is determined by the characteristics of the power delivered to the engine. The control unit measures the electrical current and voltage supplied to the motor and uses this data to calculate the torque exerted by the motor.

In a preferred embodiment, the applied torque is at least 0.001 Nm and at most 5 Nm, preferably at least 0.01 Nm and at most 4.5 Nm, preferably at least 0.1 Nm and at most 4.0 Nm, preferably at least 1.0 Nm and at most 3.5 Nm, preferably at least 1.5 Nm and at most 3.0 Nm, preferably at least 2.0 Nm and at most 2.5 Nm. Applying the correct torque is critical, especially in medical applications where an implant is secured in the body. For example, too high a torque can lead to damage to the bone or soft tissue, while too low a torque can lead to insufficient stability of the prosthesis and possible loosening or subluxation of the implant. Therefore, carefully adjusting the torque applied is an important step in the medical procedure. Using this specific screwdriver, the surgeon can precisely tailor the torque applied to the type of implant and the desired results. By precisely controlling the torque, the surgeon can reduce the risk of complications and ensure a successful procedure.

In a preferred embodiment, the screwdriver is equipped with a slip ring, which allows the signals from the torque sensor to be sent via wires to the control electronics without twisting or damaging the wiring. The slip ring also allows the torque sensor to receive power from the controller and still rotate infinitely without twisting or damaging the wiring. Alternatively, the screwdriver can be equipped with a wireless transmitter to send the signals from the torque sensor to the control unit. This can be a useful option in situations where wiring is impractical or undesirable.

It is important to note that the transcondylar pin preferably has a thread, and is connected via this thread to the condylar nut, which condylar nut is temporarily attached to the condyles of the femur. The rotation of the pin in the condylar nut causes the pin to push against the tibial plate. The exertion of a pushing force by the rotating transcondylar pin and the transcondylar nut on the tibial plate, whereby a gap between the femur and the tibia will increase and the ligaments will stretch. This allows the rotating transcondylar pin and condylar nut to exert a pushing force on the tibial plate, creating an enlarged gap between the femur and tibia. Through this opening, the ligaments and other tissues between these parts are stressed and stretched. It is essential that there is a secure connection between the transcondylar pin and the condylar nut to ensure that this force is transferred properly and accurate measurements can be made. It is therefore essential that the force is transferred correctly and that accurate measurements can be made.

In a preferred embodiment, the ligaments are stretched to a maximum of 50 mm, preferably a maximum of 48 mm, preferably a maximum of 46 mm, preferably a maximum of 44 mm, preferably a maximum of 42 mm, preferably a maximum of 40 mm, preferably a maximum of 38 mm, preferably a maximum of 36 mm, preferably a maximum of 34 mm, preferably a maximum of 32 mm, preferably a maximum of 30 mm. The stretching of the ligaments to a certain length is an important factor in determining the stability and functionality of the knee. Over-stretching can cause damage to the ligaments and other tissues in the knee, while under-stretching can result in measurements that are not accurate enough. In this preferred embodiment, the ligaments are stretched to a certain length, taking into account both the safety and accuracy of the measurements.

In a further and/or alternative embodiment, a distinction is made between the medial collateral ligament (MCL) and the lateral collateral ligament (LCL), wherein the MCL is stretched to a maximum of 80 mm, preferably a maximum of 70 mm, preferably a maximum of 60 mm, preferably a maximum of 50 mm, preferably a maximum of 40 mm, preferably a maximum of 35 mm, preferably a maximum of 30 mm and wherein the LCL is stretched to a maximum of 100 mm, preferably a maximum of 90 mm, preferably a maximum of 80 mm, preferably a maximum of 70 mm, preferably a maximum of 60 mm, preferably a maximum of 50 mm, preferably a maximum of 45 mm, preferably a maximum of 40 mm. A distinction is made between the medial collateral ligament (MCL) and the lateral collateral ligament (LCL) because these ligaments are located on different sides of the knee and have different functions in stabilizing the knee. The MCL is located on the inside of the knee and is responsible for stabilizing the knee during sideways movements. The LCL is located on the outside of the knee and is responsible for stabilizing the knee during sideways movements in the opposite direction. It is therefore desirable to differentiate between the stretching of these ligaments when determining the stability and functionality of the knee.

The method further includes converting the applied torque required to rotate the transcondylar pin into a force value applied to the ligaments. As the ligaments and other tissues begin to stretch, they put more force on the transcondylar pin. This causes the torque required to continue rotating the transcondylar pin to increase, primarily due to the increased frictional force between the transcondylar pin and the condylar nut. If the friction coefficients at the interface between the transcondylar pin and the condylar nut are known or estimated, the torque required to rotate the transcondylar pin can be converted into a force value on the ligaments.

In a next step of the method, the force value is converted into a stress value. If the cross-section of the ligaments is known or estimated, the force value can be converted into a stress value.

In a next step of the method, the gap between the femur and the tibia will be calculated. For this purpose, the rotation angle of the coupling element is measured, preferably using an encoder.

In a preferred embodiment, the screw pitch is further used to calculate the gap between the femur and tibia.

In a next step of the method, a strain value of the ligaments will be calculated using an original gap between the femur and tibia and the calculated gap between the femur and tibia.

In this way, the stress and strain can be determined at the same time during the rotation of the transcondylar pin, making it possible to visualize a stress-strain curve, which is an important source of information for the surgeon. For example, the curve can help determine the optimal stress and strain of the ligaments for a specific patient. One of the advantages of this method is that it offers the possibility to measure the stress and strain of the ligaments in real time during rotation of the transcondylar pin. This allows the surgeon to accurately determine how much force is being applied to the ligaments and whether it falls within a safe and functional range.

In a preferred embodiment, the method comprises transmitting the measured value of the applied torque and the performed rotation angle of the coupling element to an interface, the interface comprising software adapted to convert the measured value of the applied torque and the performed rotation angle of the coupling element into quantities and visualizations for display in the SS curve.

In a preferred embodiment, the SS curve based on the stress and strain values is displayed on an interface, where the SS curve of the ligaments is visualized in real time by calculating and/or measuring the stress and strain at the same time while rotating the transcondylar pin.

In a preferred embodiment, an optimal zone, and preferably an optimal point, on the SS curve is determined at which the ligament is balanced. An algorithm interprets the stress-strain curve recorded by the tool and provides the surgeon with predefined zones of laxity, as well as the optimal balanced position for the medial and lateral sides separately. The tool can automatically stop in a predefined zone, or indicate when the limit value has been reached via a signal (audio, visual, haptic and/or other). The surgeon can ignore the suggested automatic stopping point if desired and interpret the stress-strain curve according to their balancing philosophy. The surgeon may then decide to implant the femoral component in a tighter or looser position. Instead of using a manual screwdriver, where the surgeons determine this subjectively, an automated and instrumented screwdriver is used.

In a preferred embodiment, the optimal zone is determined by identifying a zone within the SS curve in which the ratio between a change in stress value and a corresponding change in strain value has an upward trend.

In an embodiment, data from the number of rotations of the screwdriver is converted into a distance by the shape/characteristics of the screw, providing a known strain distance of the ligaments. The exact stretching of the ligament can be calculated despite varying tightening of the coupling element. The torque force is measured and plotted by the sensor or motor relative to this stretching. This translates into an SS curve that determines the status of the ligament. The correct point of stretching of, or tensile force on, the ligament is determined by the fact that the force per additional application of strain (via the screw) starts to increase ever more rapidly.

Defining the optimal zone on the stress-strain curve is crucial to ensure optimal ligament balance. The optimal zone on a stress-strain curve is determined by both the lower and upper limits and will involve several criteria. For example, the optimal zone is determined by looking at the increase in force per (millimeter) strain. It is essential to establish a minimum increase in force per (millimeter) strain, as this gives an indication of the point at which the ligament begins to respond to the applied tensile force. This zone can be defined as zone 1. Defining this minimum increase in force per strain identifies the point where the ligament exhibits a steady and consistent increase in force. On the other hand, it also helps to establish a maximum increase in force per (millimeter) strain, which prevents overstretching and unnecessary stress on the ligament. This zone can be defined as zone 2.

In an embodiment, the lower limit of the optimal zone is defined by a predetermined minimum value for the rate of increase in tensile force over increase in strain distance. The predetermined minimum value, after eliminating any play between the screwdriver and the transcondylar pin, is at least 5 N/mm, preferably at least 10 N/mm, more preferably at least 15 N/mm, even more preferably at least 20 N/mm and even more preferably at least 25 N/mm. This can be calculated using a derivative of the total tensile force as a function of the total strain distance, or discretely by dividing the difference in tensile force by the difference in strain distance. Note that it is possible to take additional measures to ensure that an accidental and/or premature exceedance of the minimum value can be ignored.

In a further or alternative embodiment, the lower limit of the optimal zone is defined by a predetermined minimum value for the rate of increase in tensile force over increase in strain distance. Here, the rate of increase in tensile force per additional strain distance shall be at least equal to the minimum difference in force (expressed in Newtons) per (millimeter) strain distance, and at the same time shall not be constant over a distance greater than 0.1 mm, preferably greater than 0.2 mm, more preferably greater than 0.5 mm, even more preferably greater than 1.0 mm, even more preferably greater than 1.5 mm and even more preferably greater than 2.0 mm. This means that the ligament must have a certain degree of strength so that the tensile force increases gradually and steadily as the strain distance increases. A ligament that is too weak or too flexible would not meet these requirements and may not provide sufficient support to the joint in question.

In a further or alternative embodiment, the upper limit of the optimal zone is defined by a predetermined maximum value for the rate of increase in tensile force over increase in strain distance, which can be calculated based on a derivative of the total tensile force as a function of the total strain distance, or discretely by dividing the difference in tensile force by the difference in strain distance. This is expressed as the value of the "delta force increase" (e.g., measured in Newton) per "delta strain distance" (e.g., measured in mm). In other words, if too much of an increase in the tensile force is detected, which is necessary to obtain an increase in the strain distance, this is a sign that the ligament is maximally stretched, and further stretching would indicate that the ligament would be overloaded. The predetermined maximum value is a maximum of 15 N/mm, preferably a maximum of 20 N/mm, more preferably a maximum of 30 N/mm, even more preferably a maximum of 40 N/mm and even more preferably a maximum of 50 N/mm.

In a further or alternative embodiment, the upper limit of the optimal zone is defined by a predetermined maximum value for the rate of increase in tensile force over increase in strain distance. The predetermined maximum value here is no more than 15 N/mm. This can be calculated using a derivative of the total tensile force as a function of the total strain distance, or discretely by dividing the difference in tensile force by the difference in strain distance. This is expressed as the value of the "delta force increase" (e.g., measured in Newton) per "delta strain distance" (e.g., measured in mm). The material or system is then further stretched until a constant increase in tensile force is achieved. Initially, the tensile force is increased at an accelerated rate, and when the upper limit of the optimal zone is reached, the increase in tensile force is slowed down and eventually stabilizes to a constant value. This emphasizes the need for a controlled curvature of the curve, where the maximum acceleration of the tensile force per additional strain distance is limited. A curve that is too steep may indicate a fragile ligament that is at risk of injury under higher loads.

In a preferred embodiment, an optimal point is defined. The ligament is gradually stretched until a first zone in the strain distance is detected with a length of at least 0.50 mm and at most 1.0 mm, preferably at least 0.55 mm and at most 0.95 mm, preferably at least 0.60 and at most 0.90 mm, preferably at least 0.65 mm and at most 0.85 mm, preferably at least 0.70 mm and at most 0.80 mm and in particular about 0.75 mm, with a substantially constant increase in tensile force (constant with respect to the increase in strain distance, in other words a linear relationship between delta tensile force and delta strain distance). In other words, in the SS curve an interval in the strain distance is sought where the increase in tensile force is substantially linear in the said interval. A first linear fit is determined for this interval. To determine the ideal ligament stress, a second linear fit (linear regression) is then performed on the SS curve at a second specific interval of the ligament strain distance, namely between 1.5 mm and 2.0 mm after the first zone. Alternatively, one might consider taking a derivative at a point approximately 1.5 mm or approximately 1.75 mm or approximately 2.0 mm before the first zone. The optimal point is the intersection between the first and second linear fits of the force or stress increase. This intersection indicates where the ideal stress of the ligament can be found. It will be apparent that this optimal point can of course also be extended to an optimal zone around the optimal point.

In an alternative embodiment, the optimal position on the SS curve is indicated using a single point or zone. This corresponds to the point or zone where the ratio between a change in stress value and a corresponding change in strain value has an increasing trend.

In an alternative embodiment, the optimal position on the SS curve is determined by means of one zone. This specific zone marks the area where the ratio between a change in stress value and a corresponding change in strain value is constant.

In an alternative embodiment, the optimal position on the SS curve is determined using a single point. This specific point marks the moment when the increase in stress value per increase in strain value reaches the highest acceleration.

Additionally, it should be noted that the above version is mainly discussed with a view to the SS curve. It goes without saying that even without graphical representation, it is possible to determine the zones, fits and points/zones based on the data itself.

It should also be noted that the variation in torque force presents difficulties because it depends on the precise placement of the screw or transcondylar pin in the bone tissue. The resulting rotation angle-dependent force can be filtered out by controlling the screwdriver at variable speed. This methodology allows for a more accurate determination of the optimal balance point per patient. In addition, the application of statistical process control can also be an alternative possibility to determine the optimal zone or the optimal point on the SS curve.

According to an embodiment, the variation in the torque force depending on the positioning of the screw in the bone and the resulting rotation angle-dependent force, will be sent as feedback to the surgeon to position the screwdriver more correctly in or on the coupling element or the screw, thus reducing the variation of the measurement and thus determining the correct force point more accurately. Obtained values can also be adjusted by the surgeon to obtain a percentage stronger or looser stress depending on the patient's lifestyle.

In an alternative embodiment, this method may be adapted to be used to determine an optimal balance zone, and preferably an optimal balance point, in any possible joint. This method can also be applied and possibly adapted for other joint prostheses such as hip and shoulder implants. By measuring the stress and strain in the ligaments during the rotation of a pin, an accurate picture can be obtained of the stability and functionality of the joint prosthesis. This can help to adjust the prosthesis more precisely to the individual patient, which will lead to better balance and mobility of the joint. The method offers numerous advantages for the treatment of joint problems and can be adapted and applied to different types of joint prostheses to improve treatment and increase the patient's quality of life.

Preferably, the surgical steps for performing a TKA procedure are very important to ensure that the surgery is successful and that complications are minimized. It is important that the tool system is used correctly. As a first step, the preferred approach is used to open the knee joint. The EMS (Eeklo Modified Subvastus approach) is a preferred approach because it does not disrupt the extension mechanism of the knee. Next, the osteophytes on the medial and lateral sides of the knee are removed, as well as the osteophytes in the intercondylar notch and on the posterior side of the knee joint. The tibial incision is then performed using the surgeons' preferred alignment technique. It is recommended to make a native cut in both the sagittal and coronal planes. After the tibial incision, the transcondylar pins are placed. The patella is then returned to its original position. The medial and lateral compartments are balanced by the tool system, which will indicate when the optimal balance point has been reached. When the optimal balance point is reached, the position is fixed. When using navigation or robotics, this position can be marked and the anterior, anterior bevel, posterior and posterior bevel can be defined by this balancing. In other words, the optimal balance determines the rotation of the femoral component of the implant. When using a robot or computer assistance, the extension balance can be copied from this flexion balance, as the medial side of the joint acts as a sphere. Once all openings have been defined and all cuts have been made, the trial implants can be placed. The next steps are similar to conventional techniques and will vary depending on the specific operation being performed. It is important to follow all steps carefully to ensure that the knee surgery is successful and that there are as few complications as possible. More preferably, a cutting method as described in EP2526876 is used.

One skilled in the art will appreciate that a screwdriver according to the second aspect will be used as part of the surgical tool system according to the first aspect of the invention. Furthermore, one skilled in the art will also appreciate that the surgical tool system of the first aspect is suitable for carrying out the method of the third aspect of the invention. Consequently, each feature described in this document, both above and below, may relate to any of these three aspects of the present invention.

In the following, the invention is described on the basis of non-limiting figures which illustrate the invention, and which are not intended or should not be interpreted to limit the scope of the invention.

### DESCRIPTION OF THE DRAWINGS

The invention will now be described in more detail with reference to the following non-limiting examples or figures.

The references in the figures are:
1= screwdriver
2= coupling element
3= housing
4= ball bearing
5= torque sensor
6= slip ring
7= actuation element
8= control unit
9= battery
10=gearbox
11= motor
12= rotary encoder
13=visual indicator
14=elements for wireless communication
15= interface
16=transcondylar pin
17=condylar nut
18=tibial plate
19=knee
20=patella
21=tibia
22=fibula
23=MCL
24= LCL
25=femur
26=lateral condyle
27= medial condyle

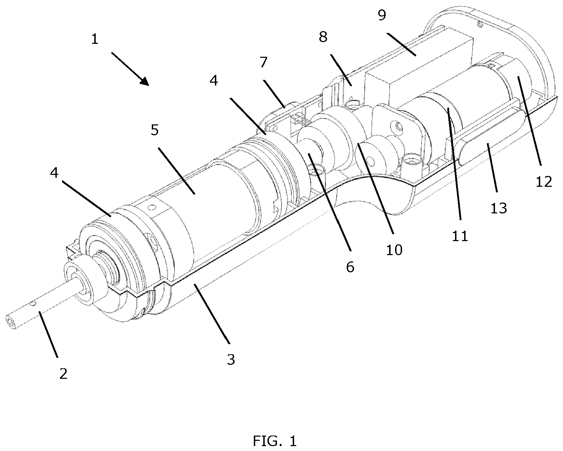

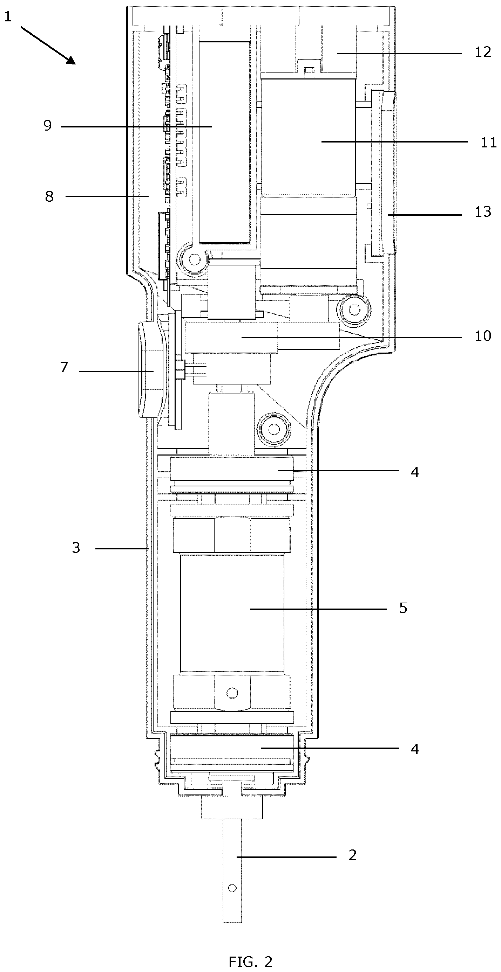

**Figures 1** **and** **2** show a detailed view of the screwdriver 1 for orthopedic procedures, where the different built-in components are clearly visible. The screwdriver 1 consists of a sterile housing 3 equipped with an actuation element 7 and a visual indicator 13. The visual indicator 13 on the screwdriver 1 allows direct interpretation of the measurement results by the surgeon.

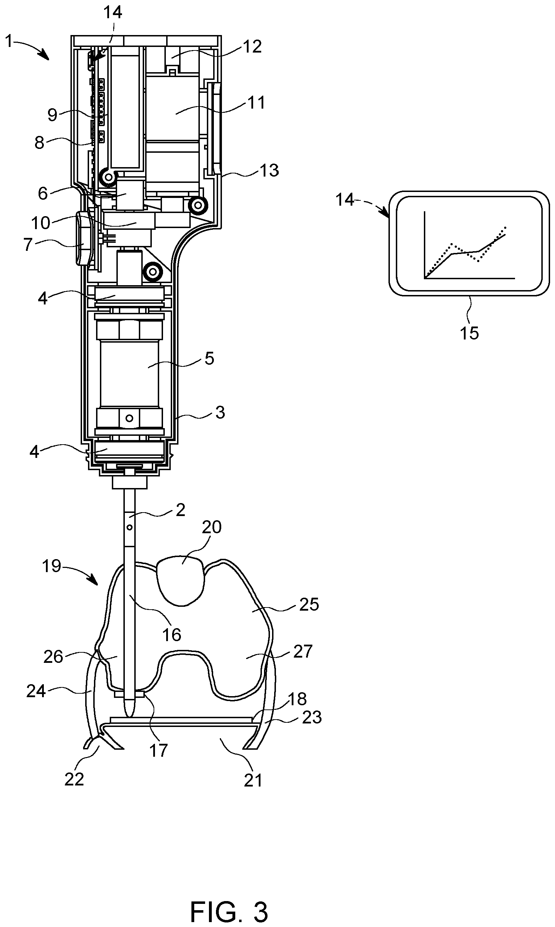

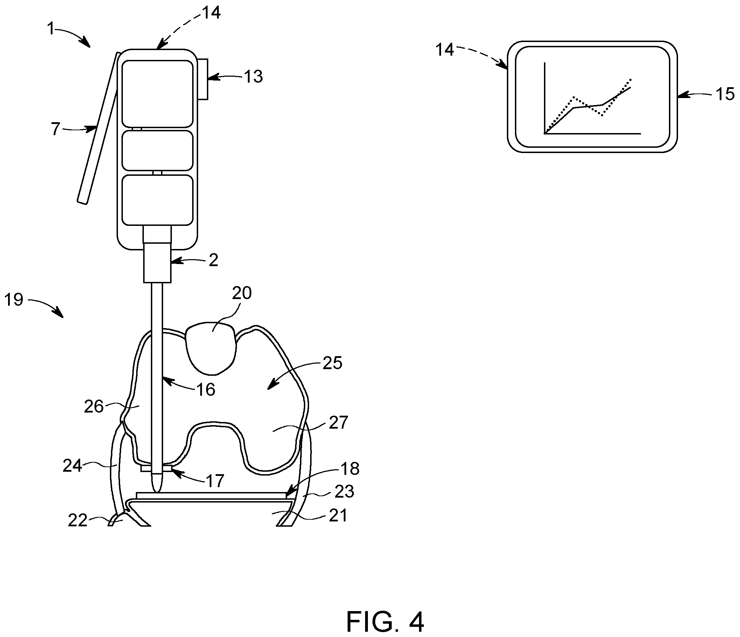

At one end of the screwdriver is the coupling element 2, which is designed to be attached to the element to be measured, ideally a transcondylar pin 16, seen in Figures 3 and 4. The coupling element 2 is provided with a hexagonal recess, which allows for an optimal connection. To perform precise measurements of the rotation angle and torque, a rotary encoder 12 and a torque sensor 5 are integrated into the screwdriver 1. The torque sensor 5 is placed between two ball bearings 4, which reduces friction and makes measurements even more accurate.

To drive the coupling element 2 and the torque sensor 5, a gearbox 10, a slip ring 6, a motor 11 and a ball bearing 4 are integrated. The motor 11 is controlled by a control unit 8 which reads and interprets the information from the torque sensor 5 and rotary encoder 12. When the actuation element 7 is activated, by pressing the button, the control unit 8 ensures that the motor 11 receives the necessary signals to perform the desired movement of the screwdriver 1, more specifically the coupling element 2 and the torque sensor 5.

The control unit 8 will send the measured information from, among others, the torque sensor 5 and rotary encoder 12 wirelessly to an interface 15 to be interpreted by medical personnel. The control unit 8 can also send relevant information to the visual indicator 13 on the screwdriver, which can be interpreted by the surgeon. The screwdriver components 1 are powered by a built-in battery 9.

**Figures 3** **and** **4** show a complete surgical tool system with the screwdriver 1 placed on transcondylar pin 16.

These figures provide a simplified representation of the knee 19 and show the anatomy of the femur 25, particularly the femoral condyles, which consist of the lateral condyle 26 and the medial condyle 27. Here you can see that the transcondylar pin 16 goes through the lateral condyle 26 of the femur 25 and that a transcondylar nut 17 is attached to the lateral condyle 26. Furthermore, it is clearly visible that the LCL 24 attaches to both the lateral condyle 26 and the fibula 22, while the MCL 23 attaches to the medial condyle 27 and to the tibia 21.

The surgical tool system also includes a tibial plate 18 which serves to protect the tibia 21 from the force generated by the transcondylar pin 16. The tibial plate 18 also provides a platform to increase the gap between femur 25 and tibia 21.

To operate the surgical tool system a control unit 8 is required, which is built into the screwdriver 1. This control unit 8 is provided with elements for wireless communication 14, so that it is connected to an interface 15, which can be, for example, a computer or mobile device. The SS curve can be displayed via this interface 15. This is an important tool for surgeons in determining the optimal balance point of the knee 19 during the procedure of placing a TKA.

Figure 3 shows the screwdriver in cross-section, which also makes the built-in components clear. These components are the same as in Figures 1 and 2.

Figure 4 shows a screwdriver where a kind of lever is used as actuation element 7. It is also noticeable that the visual indicator 13 protrudes further from the housing 3 of the screwdriver 1 than in Figures 1 and 2.

The present invention should not be construed as being limited to the embodiments described above and certain modifications or changes may be added to the examples described without having to re-evaluate the appended claims.

## Claims

1. Surgical tool system, to support prosthetic surgery of ligaments in or around a joint to determine an optimal balance point within the ligamentous complex or ligament capsule of the joint, based on and visualized in a real-time stress-strain curve (SS curve) of the ligaments, including:
- a screwdriver suitable for determining stress and strain, comprising:
i. one or more sensors, comprising at least one encoder;
ii. a coupling element, suitable for placing the screwdriver on an element to be measured;
iii. a motor suitable for driving the coupling element;
iv. a control unit, capable of determining a torque exerted by the coupling element and an executed rotation angle of the coupling element and forwarding them to an interface;
- the interface on which software runs, suitable for reading out the torque exerted by the coupling element and the executed rotation angle of the coupling element, and interpreting and forwarding it to an interface suitable for visually displaying a stress value and strain value in an SS curve, which is suitable for determining an optimal zone, and preferably an optimal point, wherein the ligament is balanced.

2. The surgical tool system according to claim 1, wherein the joint is a knee, and the optimal balance point is determined within the lateral and medial compartments of the knee.

3. The surgical tool system according to claim 2, wherein the surgical tool system further comprises:
- a condylar nut suitable for attaching to an end of the femur and suitable for receiving the transcondylar pin;
- a transcondylar pin with a screw thread that is connected via the screw thread to a condylar nut, which transcondylar pin is suitable for exerting a force on the tibial plate;
- a tibial plate suitable for protecting the tibia from the force exerted by the transcondylar pin and suitable for receiving the transcondylar pin.

4. The surgical tool system according to claim 1, wherein the joint is a hip.

5. The surgical tool system according to claim 1, wherein the joint concerns a shoulder.

6. The surgical tool system according to any of the preceding claims 1 to 5, wherein the optimal zone is determined by identifying a zone within the SS curve where the ratio of a change in stress value to a corresponding change in strain value is increasing.

7. Method for determining an optimal balance point of a ligamentous complex or ligament capsule of a joint, and preferably the lateral and medial compartment of the knee, based on and visualized in a real-time stress and strain curve (SS curve) of the ligaments for the positioning of a prosthesis, comprising:
- placing a transcondylar pin in a distal end of a femur;
- placing a condylar nut at a proximal end of the transcondylar pin;
- placing a tibial baseplate at a proximal end of the tibia;
- placing a screwdriver via a coupling element on the transcondylar pin;
- rotating the coupling element, wherein the screwdriver is driven by a motor, thereby applying a torque to the coupling element causing the transcondylar pin to rotate;
- the exertion of a pushing force by the rotating transcondylar pin and the transcondylar nut on the tibial plate, whereby a gap between the femur and the tibia will increase and the ligaments will stretch;
- converting the applied torque required to rotate the transcondylar pin into a force value applied to the ligaments;
- converting the force value into a stress value;
- calculating the gap between the femur and the tibia, comprising measuring the rotation angle of the coupling element using the encoder; and
- calculating a strain value of the ligaments using an original gap between the femur and the tibia and a calculated gap between the femur and the tibia.

8. The method according to claim 7, wherein an optimal zone, and preferably an optimal point, on the SS curve is determined at which the ligament is balanced.

9. The method according to claim 8, wherein the optimal zone is determined by identifying a zone within the SS curve where the ratio of a change in stress value to a corresponding change in strain value is increasing.

10. The method according to claim 7 or 9, the method comprising transmitting the measured value of the applied torque and the performed rotation angle of the coupling element to an interface, the interface comprising software adapted to convert the measured value of the applied torque and the performed rotation angle of the coupling element into quantities and visualizations for display in the SS curve.

11. The method according to any of the preceding claims 7 to 10, wherein the applied torque is measured by a torque sensor in the screwdriver or is determined from motor characteristics including voltage, current and power while driving the screwdriver.

12. The method according to any of the preceding claims 7 to 11, where the applied torque is at least 0.001 Nm and at most 5 Nm.

13. The method according to any of the preceding claims 7 to 12, where the SS curve based on the stress and strain values is displayed on an interface, where the SS curve of the ligaments is visualized in real time by calculating and/or measuring the stress and strain at the same time while rotating the transcondylar pin.

14. The method according to any of the preceding claims 7 to 13, wherein the ligaments are stretched to a maximum of 100 mm.

15. The method according to any of the preceding claims 7 to 14, wherein further a pitch of the screw is used to calculate the gap between the femur and the tibia.
